# EUROPEAN PATENT APPLICATION

(11) **EP 4 628 079 A1**
(43) Date of publication of application: **08.10.2025**
(21) Application number: 23896939.8
(22) Date of filing: 01.12.2023
(51) Int. Cl.: A61K 31/4365, A61K 47/38, A61K 47/32, A61K 47/36, A61K 9/20, A61P 7/02, A61P 9/10, A61P 9/00

(54) **SOLID PREPARATION CONTAINING OXIDIZED CLOPIDOGREL AND PREPARATION METHOD THEREFOR**

(30) Priority: 02.12.2022 CN 202211545401
(71) Applicant: Chengdu Shibeikang Biomedical Technology Co., Ltd., Chengdu, Sichuan 611731 (CN)
(72) Inventor: NIE, Yu, Chengdu, Sichuan 611731 (CN); YI, Zeqin, Chengdu, Sichuan 611731 (CN); ZHANG, Hai, Chengdu, Sichuan 611731 (CN); LU, Yao, Chengdu, Sichuan 611731 (CN); MOU, Xia, Chengdu, Sichuan 611731 (CN)
(74) Representative: Prinz & Partner mbB
(86) International application number: PCT/CN2023/135869
(87) International publication number: WO 2024/114797

(57) **Abstract**

The present invention provides a solid preparation containing (7aS,2'S)-2-oxo-clopidogrel and a preparation method therefor. The solid preparation is an oral tablet containing (7aS,2'S)-2-oxo-clopidogrel and pharmaceutically acceptable excipients, the excipients comprising a filler, a disintegrant, a binder, and a lubricant.

## Description

### FIELD

This application claims the priority of Chinese Patent Application No. 202211545401.6, filed with the China National Intellectual Property Administration on December 02, 2022, and titled with "SOLID PREPARATION CONTAINING OXIDIZED CLOPIDOGREL AND PREPARATION METHOD THEREFOR", which is hereby incorporated by reference.

The present application belongs to the technical field of pharmaceutical preparations, and in particular relates to a solid preparation containing (7aS, 2'S)-2-oxo-clopidogrel and a preparation method therefor.

### BACKGROUND

Clopidogrel is a first-line drug for the prevention and treatment of cardiovascular, cerebrovascular and other arterial circulatory disorders caused by high platelet aggregation. Clopidogrel bisulfate tablets are its oral dosage form. (7aS, 2'S)-2-oxo-clopidogrel is an intermediate metabolite of clopidogrel in the human body, with a structure shown in Formula I. Its chemical name is: methyl (S)-2-(2-chlorophenyl)-2-((S)-2-oxo-2,6,7,7a-tetrahydrothieno[3,2-c]pyridin-5(4H)-yl)acetate;

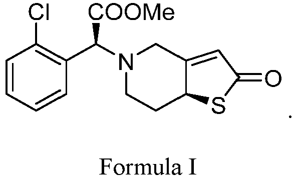

There are significant individual differences in the efficacy of clopidogrel, especially among Asians, that is, there is clopidogrel resistance (CPGR). It is due to the differences in the activity of CYP enzymes in the liver of individuals. Specifically, clopidogrel cannot be normally metabolized in the liver of some patients, and the metabolite (7aS, 2'S)-2-oxo-clopidogrel and its optical isomers cannot be produced, thus blocking the subsequent further metabolism of clopidogrel into active ingredients and failing to exert its anticoagulant effect. Direct administration of (7aS, 2'S)-2-oxo-clopidogrel can effectively avoid clopidogrel resistance.

(7aS, 2'S)-2-oxo-clopidogrel is a white or off-white crystalline powder. It is soluble in acetonitrile, slightly soluble in methanol, and almost insoluble in water. It belongs to drugs with low-solubility and high-permeability in the BCS biopharmaceutical classification system. It also has instability under high temperature, acid and alkali conditions. Therefore, it is difficult to prepare ordinary tablets with high stability. In addition, there is no report on a solid preparation of (7aS, 2'S)-2-oxo-clopidogrel that is bioequivalent to the intermediate metabolite 2-oxo-clopidogrel and the active metabolite H4 released in the human body by the commercially available clopidogrel bisulfate tablets. Therefore, it has become one of the urgent problems to be solved by those skilled in the art to research a stable solid preparation of (7aS, 2'S)-2-oxo-clopidogrel that is bioequivalent to the commercially available clopidogrel bisulfate tablets to ensure the effectiveness and safety of medication.

### SUMMARY

In view of this, the present application provides a solid preparation containing (7aS, 2'S)-2-oxo-clopidogrel for the treatment of hematological diseases, and a production method therefor. The solid preparation of (7aS, 2'S)-2-oxo-clopidogrel can continuously and stably release active ingredient and exert its therapeutic effect, and the solid preparation is stable, with reliable quality and safety.

The present application provides a solid preparation, comprising (7aS, 2'S)-2-oxo-clopidogrel and a pharmaceutically acceptable excipient; the excipient comprises a filler, a disintegrant, a binder and a lubricant.

Further, in any solid preparation as described above, a mass ratio of the disintegrant to the binder is 1 - 20:1.

Preferably, in any solid preparation as described above, a mass ratio of the disintegrant to the binder is 2.5 - 15:1.

More preferably, in any solid preparation as described above, a mass ratio of the disintegrant to the binder is 3 - 5:1.

Further, each unit dosage form of any solid preparation as described above contains the (7aS, 2'S)-2-oxo-clopidogrel in an amount of 2 mg to 9.5 mg; exemplarily but not limited to, such as 2 mg, 2.5 mg, 3 mg, 3.5 mg, 4 mg, 4.5 mg, 5 mg, 5.5 mg, 6 mg, 6.5 mg, 7 mg, 7.5 mg, 8 mg, 8.5 mg, 9 mg, and 9.5 mg.

Preferably, each unit dosage form of any solid preparation as described above contains the (7aS, 2'S)-2-oxo-clopidogrel in an amount of 2 mg to 8 mg; preferably 4 mg, 4.5 mg, 5 mg, 5.5 mg or 6 mg.

Further, any solid preparation as described above comprises the following components by weight: 1.0 - 8.0 wt% of (7aS, 2'S)-2-oxo-clopidogrel, 65.0 - 95.0 wt% of filler, 3.0 - 20.0 wt% of disintegrant, 1.0 - 6.0 wt% of binder, and 0.5 - 2.5 wt% of lubricant.

Preferably, any solid preparation as described above comprises the following components by weight: 1.0 - 8.0 wt% of (7aS, 2'S)-2-oxo-clopidogrel, 65.0 - 92.0 wt% of filler, 10.0 - 20.0 wt% of disintegrant, 1.0 - 6.0 wt% of binder, and 0.5 - 2.5 wt% of lubricant.

More preferably, any solid preparation as described above comprises the following components by weight: 2.0 - 6.0 wt% of (7aS, 2'S)-2-oxo-clopidogrel, 70.0 - 90.0 wt% of filler, 10.0 - 20.0 wt% of disintegrant, 1.0 - 6.0 wt% of binder, and 0.5 - 2.5 wt% of lubricant.

Further, any filler as described above is any one or more selected from the group consisting of mannitol, lactose, pre-gelatinized starch, sorbitol, microcrystalline cellulose, starch, powdered sugar, dextrin, and an inorganic salt; preferably two or more of pre-gelatinized starch, lactose, mannitol, microcrystalline cellulose, and sorbitol;
any disintegrant as described above is any one or more selected from the group consisting of low-substituted hydroxypropyl cellulose, crospovidone, cross-linked sodium carboxymethylcellulose, sodium carboxymethyl starch, polacrilin potassium, and dry starch; preferably one or more of low-substituted hydroxypropyl cellulose, crospovidone, cross-linked sodium carboxymethylcellulose, sodium carboxymethyl starch, and polacrilin potassium;
any binder as described above is any one or more selected from the group consisting of acacia, povidone, hydroxypropyl methylcellulose, hydroxypropyl cellulose, gelatin, sodium carboxymethyl cellulose, methyl cellulose, and copovidone; preferably one or more of povidone, hydroxypropyl methylcellulose, hydroxypropyl cellulose, sodium carboxymethyl cellulose, and copovidone; and
any lubricant as described above is any one or more selected from the group consisting of magnesium stearate, sodium stearyl fumarate, talc, a polyethylene glycol substance, and hydrogenated vegetable oil.

Further, any lactose as the filler as described above includes but is not limited to lactose monohydrate and anhydrous lactose; the inorganic salt as the filler as described above includes but is not limited to calcium sulfate, calcium hydrogen phosphate, calcium carbonate and calcium sulfate dihydrate; the polyethylene glycol substance as the lubricant as described above includes but is not limited to PEG2000, PEG4000 and PEG6000; the hydrogenated vegetable oil as the lubricant as described above includes but is not limited to hydrogenated castor oil.

Further, the solid preparation as described above comprises the following components by weight: 1.0 - 8.0 wt% of (7aS, 2'S)-2-oxo-clopidogrel, 10.0 - 35.0 wt% of pre-gelatinized starch, 30.0 - 65.0 wt% of lactose, 3.0 - 20.0 wt% of low-substituted hydroxypropyl cellulose, 1.0 - 6.0 wt% of hydroxypropyl cellulose, and 0.5 - 2.5 wt% of sodium stearyl fumarate.

Preferably, the solid preparation as described above comprises the following components by weight: 3.0 - 6.0 wt% of (7aS, 2'S)-2-oxo-clopidogrel, 10.0 - 35.0 wt% of pre-gelatinized starch, 30.0 - 65.0 wt% of lactose, 3.0 - 20.0 wt% of low-substituted hydroxypropyl cellulose, 1.0 - 6.0 wt% of hydroxypropyl cellulose, and 0.5 - 2.5 wt% of sodium stearyl fumarate.

Preferably, the solid preparation as described above comprises the following components by weight: 1.0 - 8.0 wt% of (7aS, 2'S)-2-oxo-clopidogrel, 20.0 - 35.0 wt% of pre-gelatinized starch, 40.0 - 60.0 wt% of lactose, 6.0 - 18.0 wt% of low-substituted hydroxypropyl cellulose, 1.0 - 6.0 wt% of hydroxypropyl cellulose, and 0.5 - 2.5 wt% of sodium stearyl fumarate.

Preferably, the solid preparation as described above comprises the following components by weight: 3.0 - 6.0 wt% of (7aS, 2'S)-2-oxo-clopidogrel, 20.0 - 35.0 wt% of pre-gelatinized starch, 40.0 - 60.0 wt% of lactose, 6.0 - 18.0 wt% of low-substituted hydroxypropyl cellulose, 1.0 - 6.0 wt% of hydroxypropyl cellulose, and 0.5 - 2.5 wt% of sodium stearyl fumarate.

Further, any lactose as described above includes lactose monohydrate or anhydrous lactose, preferably anhydrous lactose.

Further, in any solid preparation as described above, the (7aS, 2'S)-2-oxo-clopidogrel has a particle size distribution range of 1 µm ≤ D90 ≤ 40 µm; preferably 1 µm ≤ D90 ≤ 30 µm; more preferably 1 µm ≤ D90 ≤ 15 µm.

Preferably, in any solid preparation as described above, the (7aS, 2'S)-2-oxo-clopidogrel has a particle size distribution range of 1 µm ≤ D90 ≤ 15 µm; preferably 2 µm ≤ D90 ≤ 8 µm.

Further, any solid preparation as described above has a cumulative dissolution amount of 65% - 90% within 30 minutes in a dissolution medium of acetate buffer solution with a pH of 4.5 at a rotation speed of 50 RPM according to the second method of General Provision 0931 in Part 4 of Chinese Pharmacopoeia (2020 Edition).

Further, any solid preparation as described above has a cumulative dissolution amount of above 65% within 30 minutes, above 75 - 80% within 45 minutes, and above 85 - 90% within 90 minutes in a dissolution medium of acetate buffer solution with a pH of 4.5 at a rotation speed of 50 RPM according to the second method of General Provision 0931 in Part 4 of Chinese Pharmacopoeia (2020 Edition).

Preferably, any solid preparation as described above has a cumulative dissolution amount of above 65% within 30 minutes, above 75% within 45 minutes, and above 85% within 90 minutes in a dissolution medium of acetate buffer solution with a pH of 4.5 at a rotation speed of 50 RPM according to the second method of General Provision 0931 in Part 4 of Chinese Pharmacopoeia (2020 Edition).

More preferably, any solid preparation as described above has a cumulative dissolution amount of above 65% within 30 minutes, above 80% within 45 minutes, and above 90% within 90 minutes in a dissolution medium of acetate buffer solution with a pH of 4.5 at a rotation speed of 50 RPM according to the second method of General Provision 0931 in Part 4 of Chinese Pharmacopoeia (2020 Edition).

Further, any solid preparation as described above has a cumulative dissolution amount of above 85% within 15 minutes in a dissolution medium of hydrochloric acid buffer solution with a pH of 1.2 at a rotation speed of 50 RPM according to the second method of General Provision 0931 in Part 4 of Chinese Pharmacopoeia (2020 Edition).

Further, any solid preparation as described above can achieve bioequivalence with the active metabolite H4 metabolized from clopidogrel bisulfate tablets (Plavix, 75 mg).

Further, any solid preparation as described above can achieve bioequivalence with 2-oxo-clopidogrel metabolized from clopidogrel bisulfate tablets (Plavix, 75 mg).

Further, any solid preparation as described above is an oral solid preparation; preferably tablets, granules, capsules, soft capsules, pills, powders or pellets.

The present application further provides a method for producing any solid preparation as described above, comprising steps of:
step 1: crushing a raw material of (7aS, 2'S)-2-oxo-clopidogrel to obtain a drug powder; and
step 2: mixing the drug powder with the excipients and then compressing into tablets to obtain the solid preparation.

Further, the method for producing any solid preparation as described above comprises steps of:
step 1: weighing (7aS, 2'S)-2-oxo-clopidogrel, and crushing it to the target particle size to obtain drug powder;
step 2: weighing the excipients, pre-mixing the drug powder with any filler, then adding a previously well-mixed mixture of the remaining filler, the disintegrant and the binder, mixing uniformly, finally adding the lubricant and mixing uniformly; and
step 3: directly compressing the above-mentioned well-mixed materials into tablets using a tablet press with a tablet hardness of 3.00 - 9.00 kg to obtain the tablets of each example.

Further, the method of mixing the drug powder and excipients in the step 2 as described above includes one-time mixing and batch-by-batch mixing. It should be noted that whether the drug powder is mixed with the excipients at one time or in batches, it is acceptable as long as the condition of sufficient mixing is achieved. For example, one-time mixing is sufficient when the batch size is small, while batch-by-batch mixing has a better mixing effect when the batch size is large.

Further, in any production method as described above, the method of crushing drug includes but is not limited to jet milling.

Further, in any production method as described above, tablet production method includes but is not limited to wet granulation tableting method, dry granulation tableting method and direct powder compression method; the direct powder compression method is preferred.

Further, any other dosage form as described above is prepared by the conventional process in the art. Exemplarily, the mixed drug powder of the present application is directly or prepared into granules or pellets and then filled into capsules, soft capsules or bags to form granules, capsules, soft capsules, pills, powders or pellets, etc.

The present application further provides use of any solid preparation as described above in the treatment of hematological diseases.

Further, the hematological diseases as described above include hematological diseases of the cardiovascular and cerebrovascular systems.

Further, the hematological diseases as described above include but are not limited to acute coronary syndrome, atherosclerotic diseases, or thrombotic complications.

### Special Explanation of Terms

The term "lactose" in the present application includes hydrated lactose and anhydrous lactose, especially lactose monohydrate and anhydrous lactose. Herein, "lactose monohydrate" refers to "lactose" in the Chinese pharmacopoeia; "anhydrous lactose" has the same definition as in the Chinese pharmacopoeia. **In** the present application, the term "lactose" is used to represent the general concept to unify hydrated lactose and anhydrous lactose.

The term "filler" in the present application can also be understood as "diluent" in the field.

The excipients used in the present application are conventional excipients, preferably those included in the pharmacopoeia, and are not affected by specific differences such as the manufacturer, model, molecular weight, degree of polymerization, and configuration of the excipients. For example, mannitol 100SD, mannitol 200SD, mannitol M200, etc. are all included in the above-mentioned "mannitol".

Since (7aS, 2'S)-2-oxo-clopidogrel in the present application is an intermediate metabolite of clopidogrel, it is very difficult to achieve bioequivalence between (7aS, 2'S)-2-oxo-clopidogrel and the commercially available clopidogrel bisulfate tablets (Plavix, 75mg). In particular, it is extremely difficult to meet the equivalence of two compounds, that is, the simultaneous equivalence of the intermediate metabolite 2-oxo-clopidogrel and the active metabolite H4. The inventors of the present application have therefore conducted a great deal of exploratory research and found that different formulation compositions have a significant impact on dissolution. For example, when microcrystalline cellulose is added as a filler, although it improves the fluidity of the granules, the dissolution rate of (7aS, 2'S)-2-oxo-clopidogrel decreases slightly after tableting. Another example is that adding different amounts of disintegrants has little effect on the dissolution rate of (7aS, 2'S)-2-oxo-clopidogrel. As another example, after micronizing the raw material drug, the dissolution of (7aS, 2'S)-2-oxo-clopidogrel accelerates, but if the particle size is too small, it is prone to cause the problem of burst release, which in turn leads to too rapid drug absorption and increases the risk of bleeding; or although Cₘₐₓ is increased, AUC(0 - ∞) cannot be equivalent. In addition, after reducing the particle size of the raw material, the contact surface with the excipients increases, the drug stability decreases, and the risk of sticking to the punch increases. Therefore, conventional methods for solving the dissolution problem of poorly soluble drugs are difficult to make the solid preparation of (7aS, 2'S)-2-oxo-clopidogrel meet the requirements of continuous, stable, and complete release, and it is also difficult to balance the stability requirements of the solid preparation.

After numerous studies on the combination of specifications, particle sizes, and different formulations, the inventors finally discovered the three-dimensional dynamic balance among the appropriate specifications, particle sizes, and formulation compositions of (7aS, 2'S)-2-oxo-clopidogrel within the scope of the present application. First, the inventors of the present application unexpectedly found that adding the binder and controlling the ratio of the disintegrant to the binder can compromise the burst-release phenomenon of excessively high Cₘₐₓ at a small particle size to a certain extent, thereby reducing the risk of bleeding. Second, by limiting the ratio of the amount of the disintegrant to the binder, the stability of the solid preparation and the problem of sticking to the punch during the process can be significantly improved. This improvement is particularly remarkable for formulations with smaller particle sizes. Finally, through a large number of dissolution behavior evaluations, two *in vitro* dissolution curves that are completely bioequivalent to the commercially available clopidogrel bisulfate tablets (Plavix, 75mg) were found, which provides a scientific evaluation standard for the formulation screening of (7aS, 2'S)-2-oxo-clopidogrel.

Compared with the prior art, the present application has the following beneficial effects:
1. The solid preparation of (7aS, 2'S)-2-oxo-clopidogrel in the present application has a similar *in vitro* dissolution curve. Specifically, under the condition of pH 4.5, the cumulative dissolution amount reaches above 65% within 30 minutes, above 75-80% within 45 minutes, and above 85-90% within 90 minutes; under the condition of pH 1.2, the cumulative dissolution amount reaches above 85% within 15 minutes. The drug is released rapidly, stably, and completely.
2. The solid preparation of the present application is completely bioequivalent to the commercially available clopidogrel bisulfate tablets (Plavix, 75mg), showing good bioavailability and ensuring the effectiveness and safety of the drug. Specifically, the solid preparation of the present application and the commercially available clopidogrel bisulfate tablets (Plavix, 75mg) have bioequivalence in terms of the indicators Cₘₐₓ, AUC₍₀₋ₜ₎, and AUC_{(0-∞)} of the intermediate metabolite 2-oxo-clopidogrel and the active metabolite H4, and the Cₘₐₓ and AUC are far below the safety window, with a low risk of bleeding.
3. For the solid preparation of (7aS, 2'S)-2-oxo-clopidogrel in the present application, the excipients are commonly available, the quality of the preparation is stable, safe, and reliable, and it is of high quality and low cost.
4. The production method of the solid preparation of (7aS, 2'S)-2-oxo-clopidogrel in the present application is simple, with low production costs, and is suitable for industrial production.

### BRIEF DESCRIPTION OF DRAWINGS

FIG. 1 shows dissolution curves under the condition of pH 4.5 for Examples 1 to 8 and comparative examples.
FIG. 2 shows dissolution curves under the condition of pH 1.2 for Examples 1 to 8.
FIG. 3 shows dissolution curves under the condition of pH 4.5 for Examples 9 to 15.
FIG. 4 shows dissolution curves under the condition of pH 4.5 for Examples 16 to 27.

### DETAILED DESCRIPTION

The following will provide a further detailed description of the present application in combination with the examples and test examples. The examples and test examples of the present application are only used to illustrate the technical solution of the present application, and are not intended to limit the present application. Any equivalent substitution in the art made by those skilled in the art in accordance with the disclosed content of the present application, or all other embodiments obtained without making inventive efforts, shall fall within the protection scope of the present application. Where the specific techniques or conditions are not indicated in the examples, they shall be carried out according to the techniques or conditions described in the documents in the art or according to the product instructions. For the excipients, reagents or instruments used without indicating the manufacturer or model, they are all conventional products obtained through commercial purchase.

The drug raw material of (7aS, 2'S)-2-oxo-clopidogrel (hereinafter referred to as "this product") involved in the following test examples and examples is, without special instructions, a self-made product of Chengdu Shibeikang Biomedical Technology Co., Ltd., with a purity of over 99%. The related substances PZ5 and PZ2 mentioned in the following test examples and examples are a hydroxyl impurity and an isomer impurity respectively, and their structures are as follows. Among them, the PZ5 impurity is the main degradation impurity of (7aS, 2'S)-2-oxo-clopidogrel, and the PZ2 impurity is its main isomer impurity. As of the filing date of this application, other known impurities of this product that are known but not included in the representative samples of the quality test results of this application are absent or not detected.

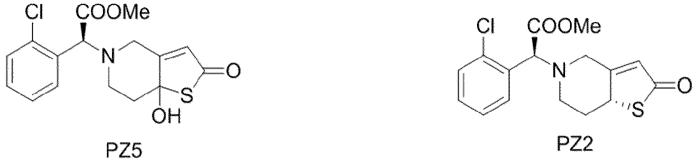

### Test Example 1: Screening of tablet formulations

(7aS, 2'S)-2-oxo-clopidogrel is extremely prone to oxidative degradation to generate the PZ5 impurity. Therefore, it is difficult to screen out a preparation with high stability simply through *in vitro* dissolution. In view of this, this test example will screen out reliable formulations through the evaluation of *in vitro* dissolution and stability of different formulations.

### 1. Screening of formulations and their preparation

On the basis of a large number of preliminary exploratory tests, in order to prove the influence of different materials and their proportions on dissolution, the representative formulations shown in the following table were screened out.

**Table 1-1 (7aS, 2'S)-2-oxo-clopidogrel tablets of different formulations**

| **Material Function** | **Component** | **Example 1** | **Exam ple 2** | **Exam ple 3** | **Exam ple 4** | **Exam ple 5** | **Exam ple 6** | **Exam ple 7** | **Exam ple** 8 |
|---|---|---|---|---|---|---|---|---|---|
| API | This product (g) | 5.0 | 5.0 | 5.0 | 5.0 | 5.0 | 5.0 | 5.0 | 5.0 |
| **Filler** | Mannitol (g) | 60.9 | 60.9 | 65.0 | 60.0 | / | / | / | 50.0 |
| | Anhydrous lactose (g) | 60.9 | 60.9 | / | / | 50.0 | 65.0 | 60.0 | / |
| | Pre-gelatinized starch (g) | / | / | 46.0 | 51.0 | 28.0 | 17.0 | 17.0 | 32.0 |
| **Disintegr ant** | Low-substituted hydroxypropyl cellulose (g) | 11.2 | 12.6 | / | / | 12.0 | 15.0 | 21.0 | / |
| | Cross-linked sodium carboxymethylcellul ose (g) | / | / | 30.0 | 30.0 | / | / | / | 7.8 |
| **Binder** | Hydroxypropyl methylcellulose (g) | 5.6 | 4.2 | / | / | / | / | / | 3.0 |
| | Hydroxypropyl cellulose (g)) | / | / | 2.0 | 1.5 | 3.0 | 1.0 | 1.3 | / |
| **Lubrican t** | Sodium stearyl fumarate (g) | / | / | 2.0 | 2.5 | 1.2 | 2.0 | 2.7 | / |
| | Hydrogenated castor oil (g) | 1.4 | 1.4 | / | / | / | / | / | 1.2 |
| **Batch Quantity (tablets)** | | 1000 | 1000 | 1000 | 1000 | 1000 | 1000 | 1000 | 1000 |
| **Disintegrant : Binder (mass ratio)** | | 2 | 3 | 15 | 20 | 4 | 15 | 16.2 | 2.6 |

Preparation method:
Step 1: (7aS, 2'S)-2-oxo-clopidogrel was weighed, and crushed to a D90 of below 15 µm to obtain drug powder;
Step 2: the excipients were weighed, the drug powder was pre-mixed with any filler, then a previously well-mixed mixture of the remaining filler, the disintegrant and the binder were added, and then mixed uniformly, and finally the lubricant was added and mixed uniformly; and
Step 3: the above-mentioned well-mixed materials were directly compressed into tablets using a tablet press with a tablet hardness of 3.00-9.00 kg to obtain the tablets of each example.

Comparative example: The method in the Comparative example was the same as the formulation B1 and its preparation method in Example 4 of Patent CN111249241A except that the active pharmaceutical ingredient thienopyridine composition was replaced with (7aS, 2'S)-2-oxo-clopidogrel of the present application.

In the formulations of Examples 1 to 8, each tablet has good fluidity, compressibility and moldability. A slight sticking phenomenon occurs in Example 7, and there is no or almost no sticking in other examples.

### 2. Dissolution determination under conditions of pH 4.5

Dissolution determination method: Samples were taken. The method was performed in a dissolution medium of acetate buffer solution with a pH of 4.5 at a rotation speed of 50 revolutions per minute according to the second method of General Provision 0931 in Part 4 of Chinese Pharmacopoeia (2020 Edition). At 5, 10, 15, 30, 45, 60, 90 and 120 minutes, the solution was filtered, and an appropriate amount of the subsequent filtrate was measured. The reference substance of (7aS, 2'S)-2-oxo-clopidogrel was weighed precisely, put into a 10 ml volumetric flask, and dissolved with acetonitrile, which was filled to the mark. 1 ml was precisely measured, put into a 100 ml volumetric flask, and filled with the dissolution medium to prepare a solution containing about 10 µg of (7aS, 2'S)-2-oxo-clopidogrel per 1 ml. The above two solutions were taken to measure the absorbance at a wavelength of 220 nm respectively according to the ultraviolet-visible spectrophotometry (General Provision 0401), and the dissolution amount of each tablet was calculated.

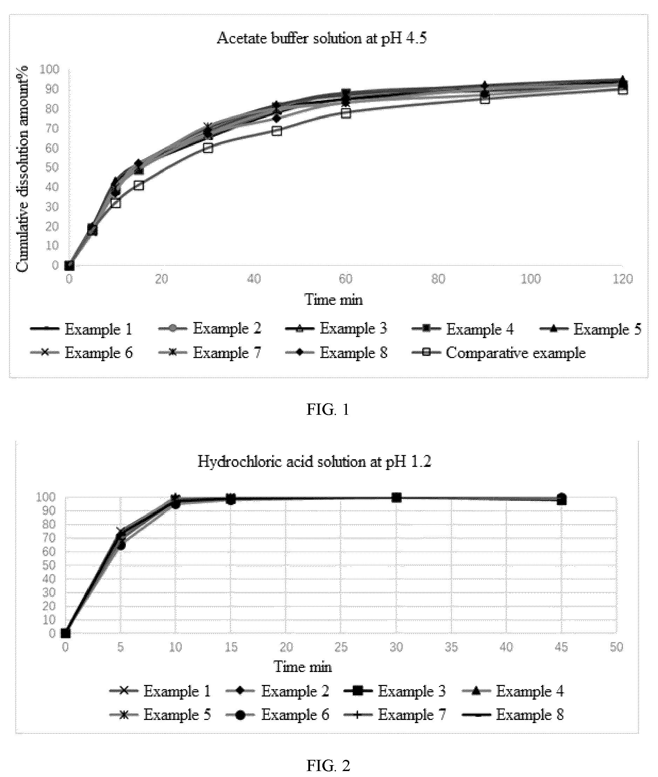

Test results: The dissolution curve results of the samples of each example in the dissolution medium of the acetate buffer with a pH of 4.5 are shown in FIG. 1. The results show that the samples of Examples 1 to 8 all had similar dissolution curves under pH 4.5 conditions, and the dissolution rate was significantly better than that of the Comparative example. Specifically, the formulations of Examples 1 to 8 had a cumulative dissolution amount of above 65% at 30 min, above 75% at 45 min, and above 85% at 90 min. Especially, Examples 2, 3, 5 and 6 dissolved faster and had better dissolution effects. It is proved that the more the amount of disintegrant is not always the better, and the type and amount of disintegrant have a limited effect on changing the disintegration rate.

### 3. Dissolution determination under conditions of pH 1.2

Dissolution determination method: Samples were taken. The method was performed according to the Dissolution and Release Determination Method (the second method of General Provision 0931, Part 4, Chinese Pharmacopoeia (2020 Edition)) in a dissolution medium of hydrochloric acid solution with a pH of 1.2 at a rotation speed of 50 revolutions per minute. At 5, 10, 15, 30 and 45 minutes, the solution was filtered, and an appropriate amount of the subsequent filtrate was measured. The reference substance of (7aS, 2'S)-2-oxo-clopidogrel was weighed precisely, put into a 10 ml volumetric flask, and dissolved with acetonitrile, which was filled to the mark. 1 ml was precisely measured, put into a 100 ml volumetric flask, and filled with the dissolution medium to prepare a solution containing about 10 µg of (7aS, 2'S)-2-oxo-clopidogrel per 1 ml. The above two solutions were taken to measure the absorbance at a wavelength of 220 nm respectively according to the ultraviolet-visible spectrophotometry (General Provision 0401), and the dissolution amount of each tablet was calculated.

Test results: The dissolution test results of the samples of each example in the dissolution medium of hydrochloric acid with a pH of 1.2 are shown in FIG. 2. The results show that Examples 1 to 8 all have good dissolution in the dissolution medium of hydrochloric acid with a pH of 1.2, that is, the cumulative dissolution amount reaches above 50% at 5 min and approaches 100% at 10 min, proving that different solid preparations of (7aS, 2'S)-2-oxo-clopidogrel have the characteristics of rapid dissolution under pH 1.2 conditions. Therefore, by comprehensively comparing with the dissolution under pH 4.5 conditions, the key index for the formulation screening of this product lied in the dissolution under pH 4.5 conditions.

### 4. Stability investigation

Investigation conditions: According to the requirements of the relevant guiding principles for stability tests, the samples of Examples 1 to 8 are investigated for accelerated stability (40°C±2°C; RH: 75%±5%).

Test results: The accelerated results of the samples of Examples 1 to 8 are shown in Tables 1-2. The results show that after 6 months of accelerated stability investigation on the samples of each example, compared with 0 day, the increment of PZ5 impurity in Examples 1, 4 and 7 was about 1%, the increment of PZ2 impurity exceeded 0.2%, and the content of the main drug decreased by about 2%. The increment of PZ5 impurity in Examples 2, 3, 5, 6 and 8 was less than 0.8%, the increment of PZ2 impurity was about 0.1%, the content of the main drug remained basically unchanged, and the maximum single impurity and other total impurities also remained basically unchanged. It is proved that Examples 2, 3, 5, 6 and 8 have a higher stability, and the quality is safer and more reliable.

**Tables 1-2 Accelerated stability test results of samples of Examples 1 to 8**

| **Sample** | **Investiga tion Time** | **Appeara nce** | **Related Substances** | | | | **Conten t (%)** |
|---|---|---|---|---|---|---|---|
| | | | **PZ5%** | **PZ2%** | **Maximum Unknown Single Impurity (%)** | **Other Total Impurities (%)** | |
| Example 1 | 0 day | White | 0.061 | 0.788 | 0.110 | 0.168 | 99.2 |
| | 6 months | Off-white | 1.071 | 0.988 | 0.140 | 0.202 | 98.1 |
| Example 2 | 0 day | White | 0.059 | 0.803 | 0.113 | 0.113 | 99.3 |
| | 6 months | Off-white | 0.811 | 0.922 | 0.134 | 0.113 | 99.3 |
| Example 3 | 0 day | White | 0.062 | 0.786 | 0.135 | 0.121 | 99.2 |
| | 6 months | Off-white | 0.823 | 0.956 | 0.151 | 0.116 | 99.1 |
| Example 4 | 0 day | White | 0.077 | 0.801 | 0.115 | 0.147 | 99.1 |
| | 6 months | Off-white | 1.092 | 1.011 | 0.140 | 0.188 | 97.7 |
| Example 5 | 0 day | White | 0.062 | 0.752 | 0.113 | 0.113 | 99.1 |
| | 6 months | Off-white | 0.689 | 0.800 | 0.112 | 0.108 | 99.2 |
| Example 6 | 0 day | White | 0.069 | 0.798 | 0.123 | 0.109 | 99.2 |
| | 6 months | Off-white | 0.601 | 0.901 | 0.145 | 0.111 | 99.2 |
| Example 7 | 0 day | White | 0.072 | 0.788 | 0.108 | 0.155 | 99.2 |
| | 6 months | Off-white | 1.098 | 1.011 | 0.122 | 0.162 | 97.1 |
| Example 8 | 0 day | White | 0.069 | 0.798 | 0.123 | 0.109 | 99.1 |
| | 6 months | Off-white | 0.601 | 0.901 | 0.145 | 0.111 | 99.0 |

### Test Example 2: Screening of particle size

The changes in different particle sizes are shown in Table 2-1 below. The formulation information and preparation method of Example 9 are the same as those of Example 7, and the formulation information and preparation method of Examples 10 to 15 are the same as those of Example 5.

**Table 2-1 Setting of different particle sizes**

| **Sample** | **Example 9** | **Example 10** | **Example 11** | **Example 12** | **Example 3** | **Example 14** | **Example 15** |
|---|---|---|---|---|---|---|---|
| D90 (µm) | 2 | 2 | 3 | 8 | 15 | 30 | 40 |

### 1. Determination of dissolution under conditions of pH 4.5

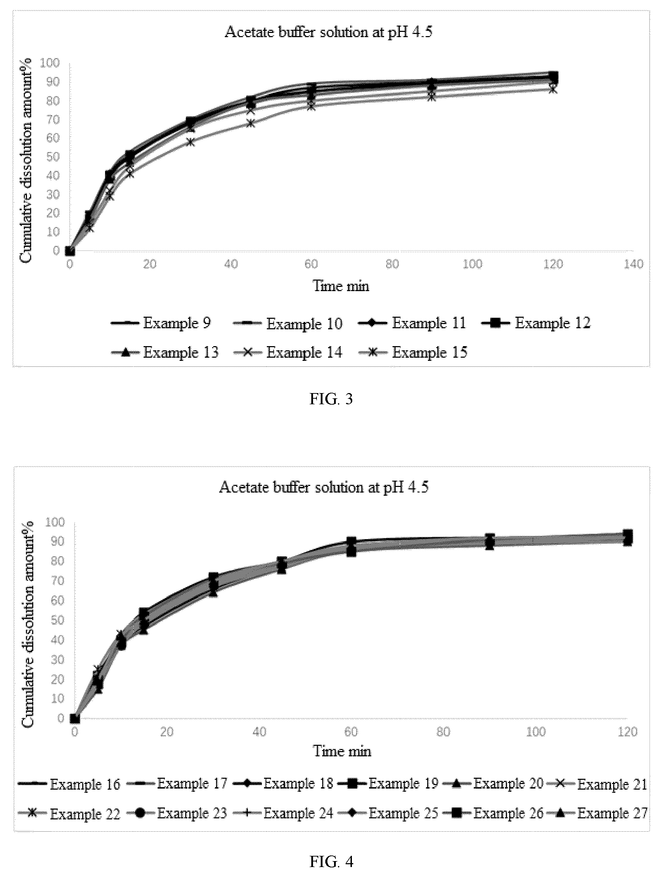

The dissolution of the above samples with different particle sizes in the acetate buffer medium with a pH of 4.5 was determined according to the method of Test Example 1, and the dissolution curves were plotted. The results are shown in FIG. 3. The results show that for Examples 9 to 14, the cumulative dissolution amount reached above 65% at 30 min, above 75% at 45 min, and above 85% at 90 min; especially for Examples 9 to 13, the cumulative dissolution amount reached about above 80% at 45 min and about above 90% at 90 min, with a faster dissolution rate and better dissolution effect. However, for Example 15, the cumulative dissolution amount was less than 60% at 30 min, less than 70% at 45 min, and less than 85% at 90 min, with a relatively slow dissolution rate, proving that the larger the particle size in the formulation, the slower the dissolution.

### 2. Stability investigation

The stability of the samples of Examples 9 to 15 was investigated according to the "Stability Investigation" under Test Example 1. The statistical results of the test are shown in the following table.

**Table 2-2 Accelerated stability test results of samples of Examples 9 to 15**

| **Sample** | **Investigation Time** | **Appearan ce** | **Related Substances** | | | | **Content (%)** |
|---|---|---|---|---|---|---|---|
| | | | **PZ5 %** | **PZ2 %** | **Maximu m Unknow n Single Impurit y (%)** | **Other Total Impurities (%)** | |
| Example 9 | 0 day | White | 0.062 | 0.815 | 0.118 | 0.120 | 99.0 |
| | 6 months | Off-white | 1.092 | 1.015 | 0.121 | 0.111 | 97.4 |
| Example 10 | 0 day | White | 0.062 | 0.801 | 0.113 | 0.115 | 99.1 |
| | 6 months | Off-white | 0.603 | 0.902 | 0.124 | 0.103 | 98.6 |
| Example 11 | 0 day | White | 0.045 | 0.721 | 0.126 | 0.123 | 99.2 |
| | 6 months | Off-white | 0.553 | 0.815 | 0.149 | 0.129 | 99.0 |
| Example 12 | 0 day | White | 0.057 | 0.695 | 0.122 | 0.119 | 99.3 |
| | 6 months | Off-white | 0.596 | 0.776 | 0.153 | 0.123 | 99.3 |
| Example 13 | 0 day | White | 0.044 | 0.735 | 0.122 | 0.119 | 99.3 |
| | 6 months | Off-white | 0.591 | 0.847 | 0.153 | 0.123 | 99.4 |
| Example 14 | 0 day | White | 0.048 | 0.873 | 0.146 | 0.125 | 99.3 |
| | 6 months | Off-white | 0.563 | 0.948 | 0.167 | 0.130 | 99.3 |
| Example 15 | 0 day | White | 0.059 | 0.823 | 0.141 | 0.130 | 99.0 |
| | 6 months | Off-white | 0.571 | 0.998 | 0.154 | 0.122 | 98.9 |

The test results in the above table show that after 6 months of accelerated stability investigation on the samples of each example, compared with 0 day, for Example 9, the increment of PZ5 impurity was about 1%, the increment of PZ2 impurity was about 0.2%, and the content of the main drug decreased by 1.6%. For Examples 10 to 15, the maximum increment of PZ5 impurity was about 0.5%, the maximum increment of PZ2 impurity was about 0.1%, the maximum decrease in the content of the main drug did not exceed 0.5%, and the contents of the maximum unknown single impurity and other total impurities were basically stable. It is proved that Examples 10 to 15 have a higher stability, and especially for Examples 11 to 15, the content of the main drug remained basically unchanged, showing a better stability.

In summary, the above test results further prove that although reducing the particle size can increase the dissolution rate, it will increase the risk of impurity degradation. By adjusting the formulation ratio, especially when the mass ratio of the disintegrant to the binder in the formulation reaches 4:1, it is beneficial to improve the stability of the solid preparation, especially for the formulation with a small particle size, the stabilizing effect is significant. Further considering the stability investigation results of Test Example 1, it is proved that when the mass ratio of the disintegrant to the binder meets 2.5 - 15:1, it is beneficial to improving the stability of the solid preparation.

### Test Example 3: Screening of amount of the formulations

In order to verify the amount tolerance of each formulation and screen the appropriate amount for bioequivalence, an amount screening study was carried out. The formulations with different amount changes are shown in Examples 16 to 27 below. The preparation method of each example was the same as that of Test Example 1.

**Table 3-1 Formulations with different amounts**

| **Component** | **Example 16** | **Example 17** | **Example 18** | **Example 19** | **Example 20** | **Example 21** | **Example 22** |
|---|---|---|---|---|---|---|---|
| This Product (g) | 2.0 | 4.0 | 6.0 | 8.0 | 10.0 | 4.0 | 6.0 |
| Anhydrous lactose (g) | 130.0 | *55.0* | 65.0 | 30.0 | 41.0 | 65.0 | 30.0 |
| Pre-gelatinized starch (g) | 59.0 | 21.0 | 10.0 | 35.0 | 30.0 | 26.5 | 35.0 |
| Low-substituted hydroxypropyl cellulose (g) | 6.0 | 14.5 | 13.0 | 20.0 | 15.0 | 3.0 | 20.0 |
| Hydroxypropyl cellulose (g) | 2.0 | 3.0 | 4.0 | 6.0 | 2.0 | 1.0 | 6.0 |
| Sodium stearyl fumarate (g) | 1.0 | 2.5 | 2.0 | 1.0 | 2.0 | 0.5 | 2.5 |
| Batch quantity (tablets) | 1000 | 1000 | 1000 | 1000 | 1000 | 1000 | 1000 |

### Example 23: 3 mg tablet formulation

| **Component** | **Amount (g)** |
|---|---|
| This product | 3.0 |
| Sorbitol | 43.0 |
| Mannitol | 31.0 |
| Polacrilin potassium | 20.0 |
| Copovidone | 2.0 |
| Magnesium stearate | 1.0 |
| Batch quantity (Tablets) | 1000 |

### Example 24: 5 mg tablet formulation

| **Component** | **Amount (g)** |
|---|---|
| This product | 5.0 |
| Lactose monohydrate | 50.4 |
| Pre-gelatinized starch | 28.2 |
| Sodium carboxymethyl starch | 12.2 |
| Sodium carboxymethyl cellulose | 3.0 |
| Talc | 1.2 |
| Batch quantity (Tablets) | 1000 |

### Example 25: 6 mg tablet formulation

| **Component** | Amount **(g)** |
|---|---|
| This product | 6.0 |
| Microcrystalline cellulose | 30.2 |
| Sorbitol | 35.2 |
| Crospovidone | 20.1 |
| Povidone | 6.0 |
| PEG6000 | 2.5 |
| Batch quantity (Tablets) | 1000 |

### Example 26: 4 mg tablet formulation

| **Component** | Amount **(g)** |
|---|---|
| This product | 4.0 |
| Anhydrous lactose | 35.0 |
| Microcrystalline cellulose | 30.0 |
| Pre-gelatinized starch | 26.5 |
| Low-substituted hydroxypropyl cellulose | 3.0 |
| Hydroxypropyl cellulose | 1.0 |
| Sodium stearyl fumarate | 0.5 |
| Batch quantity (Tablets) | 1000 |

### Example 27: 6 mg tablet formulation

| **Component** | Amount **(g)** |
|---|---|
| This product | 6.0 |
| Mannitol | 30.2 |
| Lactose monohydrate | 10.0 |
| Pre-gelatinized starch | 25.2 |
| Cross-linked sodium carboxymethylcellulose | 20.1 |
| Copovidone | 6.0 |
| Magnesium stearate | 2.5 |
| Batch quantity (Tablets) | 1000 |

### 1. Dissolution determination under conditions of pH 4.5

The dissolution of the tablets prepared in the above-mentioned Examples 16 to 27 was determined in the acetate buffer medium with a pH of 4.5 according to the method of Test Example 1. The results are shown in FIG. 4. The results show that for the samples of Examples 16 to 19 and 21 to 27, the cumulative dissolution amount reached above 65% at 30 minutes, above 75% at 45 minutes, and above 90% at 90 minutes, and the dissolution curves were similar. However, for Example 20, the cumulative dissolution amount was 64% at 30 minutes, reached 76% at 45 minutes, and reached 88% at 90 minutes, with a slightly slower dissolution. The possible reason is that the large amount affects the dissolution effect, so it is recommended to control the amount below 10 mg.

### 2. Stability investigation

The test was conducted according to the "Stability investigation" under Test Example 1, and the statistical results are shown in the following table.

**Table 3-2 Accelerated stability test results of solid preparations in Examples 16 to 27**

| **Sample** | **Investigatio n Time** | **Appeara nce** | **Related Substances** | | | | **Conten t %** |
|---|---|---|---|---|---|---|---|
| | | | **PZ5%** | **PZ2%** | **Maximum Unknown Single Impurity %** | **Other Total Impurities %** | |
| Example 16 | 0 day | White | 0.050 | 0.973 | 0.131 | 0.125 | 99.1 |
| | 6 months | Off-white | 0.543 | 1.023 | 0.130 | 0.116 | 99.0 |
| Example 17 | 0 day | White | 0.055 | 0.903 | 0.126 | 0.125 | 99.0 |
| | 6 months | Off-white | 0.558 | 1.003 | 0.131 | 0.128 | 99.0 |
| Example 18 | 0 day | White | 0.060 | 0.866 | 0.142 | 0.130 | 99.1 |
| | 6 months | Off-white | 0.600 | 0.991 | 0.153 | 0.115 | 99.1 |
| Example 19 | 0 day | White | 0.043 | 0.879 | 0.132 | 0.113 | 99.1 |
| | 6 months | Off-white | 0.530 | 0.986 | 0.150 | 0.116 | 99.0 |
| Example 20 | 0 day | White | 0.041 | 0.950 | 0.132 | 0.122 | 99.0 |
| | 6 months | Off-white | 0.866 | 1.001 | 0.143 | 0.125 | 98.0 |
| Example 21 | 0 day | White | 0.041 | 0.950 | 0.132 | 0.122 | 99.0 |
| | 6 months | Off-white | 0.566 | 1.001 | 0.143 | 0.125 | 99.0 |
| Example 22 | 0 day | White | 0.049 | 0.901 | 0.120 | 0.126 | 99.0 |
| | 6 months | Off-white | 0.556 | 0.992 | 0.146 | 0.125 | 99.0 |
| Example 23 | 0 day | White | 0.053 | 0.856 | 0.128 | 0.128 | 99.2 |
| | 6 months | Off-white | 0.586 | 0.912 | 0.166 | 0.145 | 98.7 |
| Example 24 | 0 day | White | 0.050 | 0.801 | 0.135 | 0.106 | 99.1 |
| | 6 months | Off-white | 0.622 | 0.953 | 0.151 | 0.117 | 98.8 |
| Example 25 | 0 day | White | 0.055 | 0.826 | 0.132 | 0.107 | 99.2 |
| | 6 months | Off-white | *0.555* | 0.901 | 0.139 | 0.110 | 98.8 |
| Example 26 | 0 day | White | 0.051 | 0.811 | 0.135 | 0.106 | 99.1 |
| | 6 months | Off-white | 0.560 | 0.906 | 0.140 | 0.105 | 98.8 |
| Example 27 | 0 day | White | 0.052 | 0.802 | 0.137 | 0.108 | 99.0 |
| | 6 months | Off-white | 0.575 | 0.822 | 0.136 | 0.115 | 98.5 |

The test results show that after 6 months of accelerated stability investigation, compared with 0 day, for Example 9, the increment of PZ5 in the related substances of each example sample was about 0.5% (not exceeding 1.0%), the increment of PZ2 did not exceed 0.2%, the maximum unknown single impurity and other total impurities basically remained unchanged, and the change in content did not exceed 1.0%. In particular, the change in content of Examples 16 to 19 and 21 to 27 did not exceed 0.5%, proving that the formulation within the scope of the present application has good quality stability.

### Test Example 4: Bioequivalence study in healthy adults

### 1. Tested drugs

Example 6, Example 10, Example 13, Example 21, Example 22.

### 2. Administration on an empty stomach

Each tested drug was tested on 30 healthy adult volunteers (at least one-third of them were female), and they were randomly divided into 2 groups with 15 people in each group. The test was divided into 2 stages. The administration grouping was as follows: the test preparation or the reference preparation (Plavix, 75 mg) was randomly taken orally on an empty stomach, as shown in Table 4-1.

**Table 4-1 Grouping for administration on an empty stomach**

| | **Administration Grouping** | **Group A** | **Group B** |
|---|---|---|---|
| | Sequence | T-R | R-T |
| Administration on an empty stomach | The first cycle | T | R |
| | Washout period | ≥4 days | |
| | The second cycle | R | T |

A single-center, single-dose, randomized, open-label, two-sequence, two-period, crossover design method was adopted for single-dose administration (the washout period was ≥ 4 days). The 30 selected healthy volunteers were randomly divided into Group A and Group B, with 15 people in each group. Volunteers randomly took the Example preparation (test preparation: T) or the reference preparation (Plavix: R) orally on an empty stomach. The active thiol metabolite H4 of clopidogrel and 2-oxo-clopidogrel in the plasma were measured.

### 3. Blood sample collection

On the morning of the test day, an indwelling needle was placed in the forearm vein of each subject before drug administration (it could be retained until 12 hours after drug administration). 3 ml of blank blood sample was collected 0.5 hours before drug administration. Then, 3 ml of venous blood was collected at 0.25 h, 0.5 h, 0.75 h, 1.0 h, 1.5 h, 2 h, 2.5 h, 3.0 h, 4.0 h, 6.0 h, 8.0 h, 10 h, 12 h, and 24 h (the next day) respectively after drug administration, a total of 15 times (including the blank blood sample). At each blood collection point, 2 tubes of whole blood were collected. One tube was placed in a vacuum blood-collection tube pre-added with a derivatization reagent and EDTA-K2 anticoagulant. After mixing, it was placed on ice for 10 minutes and then centrifuged. The plasma sample was transferred into a cryotube labeled with identification information. The other tube was placed in a vacuum blood-collection tube pre-added with TCEP antioxidant and EDTA-K2 anticoagulant. After mixing, it was cooled on ice, shaken well, and then centrifuged. The plasma sample was also transferred into a cryotube labeled with identification information. The time from whole-blood collection to freezing in a refrigerator at -60 to -90°C should be less than 60 minutes.

### 4. Drug concentration determination and data analysis

### Drug concentration determination

The concentrations of the derivative of the active thiol metabolite H4 of clopidogrel and 2-oxo-clopidogrel in plasma were determined by LC-MS/MS.

### Pharmacokinetic parameter statistics and analysis

The blood-drug concentration data of each subject at each time point after single-dose administration were calculated, and the drug-time curve was plotted. The mean blood-drug concentrations of subjects at each time point after taking the test preparation and the reference preparation orally were calculated respectively, and the mean drug-time curve was plotted. The software WinNonlin 7.5 was used to process the data of blood-drug concentration changes over time after single-dose administration. Pharmacokinetic parameters such as AUC (calculated by statistical moments), Cₘₐₓ (measured value), and Tₘₐₓ (measured value) were obtained. The bioavailability (F value) was calculated, and a bioequivalence analysis was performed simultaneously. Statistical analysis was carried out using analysis of variance and two-one-sided t-test, and the bioequivalence was judged based on the 1-α confidence interval.

### Pharmacodynamic parameter statistics and analysis

Statistical analysis was performed using SAS 9.1 statistical software. For the main efficacy index of the non-inferiority test, a one-sided t-test was used, and a P-value < 0.025 was considered statistically significant; for the remaining indices, a two-sided test was used, and a P-value < 0.05 was considered statistically significant. For quantitative indices, the mean, standard deviation, median, maximum, and minimum values are listed.

### 5. Test Results

After Plavix enters the human body, it is first metabolized by CYP enzymes to form 2-oxo-clopidogrel, and then further metabolized by CYP enzymes to produce the active metabolite H4. After (7aS, 2'S) 2-oxo-clopidogrel enters the body, it is rapidly converted to 2-oxo-clopidogrel, which is then metabolized by CYP enzymes to produce the active metabolite H4. Here, 2-oxo-clopidogrel is a racemate, which contains the optical isomer of (7aS, 2'S) 2-oxo-clopidogrel.

The bioequivalence results of each test preparation and reference preparation (Plavix^{®}) on an empty stomach are shown in Table 4-2 below.

**Table 4-2 Bioequivalence results of each test preparation and reference preparation (Plavix^{®})**

| **Test preparation** | **Detection Index** | **Cₘₐₓ** | | **AUC₍₀₋ₜ₎** | | **AUC_{(0-∞)}** | |
|---|---|---|---|---|---|---|---|
| | | **GMR%** | **90%CI** | **GMR%** | **90%CI** | **GMR%** | **90%CI** |
| Example 6 | 2-oxo-clopidogrel | 114.3 | 110.3-119.5% | 112.7 | 108.5-121.3% | 113.6 | 109.4-122.5% |
| | H4 | 116.6 | 112.4-121.6% | 114.2 | 113.1-119.3% | 114.8 | 114.5-121.3% |
| Example 10 | 2-oxo-clopidogrel | 118.8 | 116.3-122.7% | 116.1 | 114.3-123.4% | 117.3 | 115.6-124.2% |
| | H4 | 117.9 | 114.2-119.3% | 113.5 | 116.2-124.3% | 115.6 | 117.3-123.4% |
| Example 13 | 2-oxo-clopidogrel | 82.6 | 81.6-97.6% | 84.3 | 83.6-98.1% | 85.5 | 85.3-99.5% |
| | H4 | 84.2 | 84.6-99.2% | 86.7 | 86.6-103.5% | 87.5 | 87.3-105.4% |
| Example 21 | 2-oxo-clopidogrel | 88.5 | 90.4-103.5% | 90.1 | 92.8-105.1% | 91.4 | 90.3-102.5% |
| | H4 | 90.4 | 91.3-106.5% | 92.9 | 93.8-109.2% | 93.4 | 91.6-112.6% |
| Example 22 | 2-oxo-clopidogrel | 120.5 | 115.3-122.8% | 118.2 | 111.5-120.6% | 119.3 | 113.4-121.8% |
| | H4 | 121.9 | 114.2-123.6% | 120.2 | 115.4-122.3% | 121.5 | 114.5-122.1% |

| | | | | | | | |
|---|---|---|---|---|---|---|---|
| Note: In the above table, t is 24 h. | | | | | | | |

The above statistical results show that the 90% confidence intervals of Cₘₐₓ, AUC(0-t), and AUC (0-∞) of the two detection indices (2-oxo-clopidogrel and H4) of the samples in Examples 6, 10, 13, 21, and 22 of the present application are all between 80% and 125% of the reference preparation (Plavix^{®}), proving that the examples of the present application are bioequivalent to the reference preparation. Based on this, it can be reasonably expected that formulations with similar *in vitro* dissolution curves to Examples 6, 10, 13, 21, and 22 can achieve bioequivalence with Plavix^{®}.

The above is only the preferred embodiment of the present application. It should be noted that for those skilled in the art, without departing from the technical principle of the present application, various modifications to these embodiments can be made, and these modifications should also be regarded as the scope protected by the present application.

## Claims

1. A solid preparation, comprising (7aS, 2'S)-2-oxo-clopidogrel and a pharmaceutically acceptable excipient, and the excipient comprises a filler, a disintegrant, a binder, and a lubricant.

2. The solid preparation according to claim 1, wherein a mass ratio of the disintegrant to the binder is 2.5 - 15:1.

3. The solid preparation according to claim 1 or 2, wherein each unit dosage form of the solid preparation contains the (7aS, 2'S)-2-oxo-clopidogrel in an amount of below 10 mg, such as 2 - 9.5 mg; preferably 4 mg, 4.5 mg, 5 mg, 5.5 mg, or 6 mg.

4. The solid preparation according to claim 1 or 2, wherein the solid preparation comprises 1.0 - 8.0 wt% of (7aS, 2'S)-2-oxo-clopidogrel, 65.0 - 95.0 wt% of the filler, 3.0 - 20.0 wt% of the disintegrant, 1.0 - 6.0 wt% of the binder, and 0.5 - 2.5 wt% of the lubricant.

5. The solid preparation according to claim 1 or 2, wherein
the filler is any one or more selected from the group consisting of mannitol, lactose, pre-gelatinized starch, sorbitol, microcrystalline cellulose, starch, powdered sugar, dextrin, and inorganic salts; preferably two or more of pre-gelatinized starch, lactose, mannitol, microcrystalline cellulose, and sorbitol;
the disintegrant is any one or more selected from the group consisting of low-substituted hydroxypropyl cellulose, crospovidone, cross-linked sodium carboxymethylcellulose, sodium carboxymethyl starch, polacrilin potassium, and dry starch; preferably one or more of low-substituted hydroxypropyl cellulose, crospovidone, cross-linked sodium carboxymethylcellulose, sodium carboxymethyl starch, and polacrilin potassium;
the binder is any one or more selected from the group consisting of acacia, povidone, hydroxypropyl methylcellulose, hydroxypropyl cellulose, gelatin, sodium carboxymethyl cellulose, methyl cellulose, and copovidone; preferably one or more of povidone, hydroxypropyl methylcellulose, hydroxypropyl cellulose, sodium carboxymethyl cellulose, and copovidone; and
the lubricant is any one or more selected from the group consisting of magnesium stearate, sodium stearyl fumarate, talc, a polyethylene glycol substance, and hydrogenated vegetable oil.

6. The solid preparation according to claim 1 or 2, wherein the solid preparation comprises 1.0 - 8.0 wt% of (7aS, 2'S)-2-oxo-clopidogrel, 10.0 - 35.0 wt% of pre-gelatinized starch, 30.0 - 65.0 wt% of lactose, 3.0 - 20.0 wt% of low-substituted hydroxypropyl cellulose, 1.0 - 6.0 wt% of hydroxypropyl cellulose, and 0.5 - 2.5 wt% of sodium stearyl fumarate.

7. The solid preparation according to any one of claims 1-6, wherein the (7aS, 2'S)-2-oxo-clopidogrel has a particle size distribution range of 1 µm ≤ D90 ≤ 40 µm; preferably 1 µm ≤ D90 ≤ 30 µm; more preferably 1 µm ≤ D90 ≤ 15 µm.

8. The solid preparation according to any one of claims 1-7, wherein the solid preparation has a cumulative dissolution amount of above 65% within 30 minutes, above 75 - 80% within 45 minutes, and above 85 - 90% within 90 minutes in a dissolution medium of acetate buffer solution with a pH of 4.5 at a rotation speed of 50 RPM according to the second method of General Provision 0931 in Part 4 of Chinese Pharmacopoeia (2020 Edition); or
the solid preparation has a cumulative dissolution amount of above 85% within 15 minutes in a dissolution medium of hydrochloric acid buffer solution with a pH of 1.2 at a rotation speed of 50 RPM according to the second method of General Provision 0931 in Part 4 of Chinese Pharmacopoeia (2020 Edition).

9. The solid preparation according to any one of claims 1-7, wherein the solid preparation is an oral solid preparation, preferably tablets, granules, capsules, soft capsules, pills, powders, or pellets.

10. A method for producing the solid preparation according to any one of claims 1-7, comprising steps of:
step 1: crushing a raw material of (7aS, 2'S)-2-oxo-clopidogrel to obtain a drug powder; and
step 2: mixing the drug powder with the excipients and then compressing into tablets to obtain the solid preparation.
